# Europäisches Patentamt

# European Patent Office

# Office européen des brevets

(19)

(11) Veröffentlichungsnummer: **0 176 691**
**B1**

(12) ## EUROPÄISCHE PATENTSCHRIFT

(45) Veröffentlichungstag der Patentschrift:
**01.06.88**

(51) Int. Cl.⁴: **C 07 C 61/39**, C 07 C 51/567

(21) Anmeldenummer: **85109480.5**

(22) Anmeldetag: **27.07.85**

(54) **Verfahren zur Umsetzung von Maleinsäureanhydrid mit Vinylbenzolen.**

(30) Priorität: **27.09.84 DE 3435429**

(43) Veröffentlichungstag der Anmeldung:
**09.04.86 Patentblatt 86/15**

(45) Bekanntmachung des Hinweises auf die Patenterteilung:
**01.06.88 Patentblatt 88/22**

(84) Benannte Vertragsstaaten:
**AT BE DE FR GB IT NL**

(56) Entgegenhaltungen:
**US - A - 3 410 876**

(73) Patentinhaber: **HÜLS AKTIENGESELLSCHAFT, - RSP Patente / PB 15 - Postfach 13 20, D-4370 Marl 1 (DE)**

(72) Erfinder: **Olbrich, Jürgen, Dr., Am Gecksbach 64, D-4270 Dorsten 11 (DE)**
Erfinder: **Dörffel, Jörg, Dr., Münsterlandstrasse 45, D-4370 Marl (DE)**

## Beschreibung

Gegenstand der Erfindung ist ein Verfahren zur Umsetzung von Maleinsäureanhydrid mit ggf. kernsubstituierten (Di)Vinylbenzolen im Molverhältnis 2 : 0,7 bis 1,3 zu Dianhydrid bei Temperaturen im Bereich von 90 bis 140°C in Gegenwart eines Lösemittels und ggf. substituiertem Hydrochinon unter Durchleiten eines sauerstoffhaltigen Stickstoffstromes durch das Reaktionsgemisch und wobei das Maleinsäureanhydrid in einer 40- bis 90gew.%igen Lösung vorliegt.

Die Umsetzung von Maleinsäureanhydrid mit Vinylbenzolen zu Dianhydriden ist seit langem bekannt. Um eine Polymerisation zu vermeiden, wird unter Durchleiten eines oxidierenden Gases gearbeitet. U.a. wird hierfür Stickstoff mit einem maximalen Sauerstoffanteil von 5% (US-PS 3 769 304) beschrieben. Es wird zwar nicht ausdrücklich erläutert, warum auf keinen Fall der Sauerstoffanteil höher liegen darf, jedoch der Hinweis, der Gasstrom dürfe nicht entflammbar sein, bietet eine mögliche Erklärung.

Andererseits ist aus Journ. Am. Chem. Soc. 78, 1017 (1956) bzw. 80, 2465 (1958) bekannt, dass Styrol bei höheren Sauerstoffgehalten der Gasatmosphäre zu Styroloxid oder -peroxid oxidiert wird.

Aufgabe der Erfindung war es, ein Verfahren des Standes der Technik zu vereinfachen, ohne dass die Reaktion in Richtung oxidiertes Styrol abläuft.

Unerwarteter Weise wurde gefunden, dass die Reaktion auch dann noch möglich ist, wenn pro Mol Vinylgruppe 2 bis 20 l/h Luft (gemessen bei Normalbedingungen: 20°C/1 bar) durch das Reaktionsgemisch durchgeleitet wird.

In einer bevorzugten Ausführungsform werden 3 bis 20 l/h Luft hindurchgeleitet.

Neben dem Maleinsäureanhydrid werden ggf. kernsubstituierte Vinyl- und Divinylbenzole eingesetzt, bei letzteren können die Vinylgruppen in o-, m- bzw. p-Stellung angeordnet sein. Für die Kernsubstitution kommen in erster Linie Alkylreste mit 1 bis 7 C-Atomen in Frage. Neben den reinen Isomeren können auch Isomerengemische eingesetzt werden.

Maleinsäureanhydrid und das jeweilige Vinylbenzol werden im Molverhältnis 2 : 0,7 bis 2 : 1,3, vorzugsweise 2 : 0,95 bis 2 : 1,1, eingesetzt. Eine Erhöhung des Maleinsäureanteils in der Mischung über das angegebene Verhältnis hinaus führt zur Bildung von unerwünschten cycloaliphatischen Verbindungen. Die Erniedrigung dieses Anteils führt zur Bildung von Polyvinylbenzolen.

Als weiterer Inhibitor wird der Mischung Hydrochinon hinzugefügt. Anstelle von Hydrochinon können selbstverständlich auch seine Derivate eingesetzt werden, wie z.B. Tolylhydrochinon, Ethylhydrochinon, Isopropylhydrochinon, Di-tert.-butylhydrochinon o.ä. Die Konzentration dieser Produkte betägt üblicherweise 0,4 bis 1 Gew.-%, bezogen auf das Gemisch Maleinsäureanhydrid/Vinylbenzol.

Die Reaktionstemperaturen liegen im Bereich von 90 bis 140°C, vorzugsweise im Bereich von 105 bis 125°C.

Die Reaktion wird in Lösung durchgeführt. Hierfür eignen sich alle inerten Lösemittel mit einem Siedepunkt im Bereich von 100 bis 180°C; als Beispiel seien entsprechende (cyclo)aliphatische Lösemittel, wie z.B. Octan, Decan, Dimethylhexan, Dimethylcyclohexan, Methylcyclohexan, oder aromatische Lösemittel, wie z.B. Toluol, Xylol, Benzol genannt.

Die Konzentration von Maleinsäureanhydrid im Lösemittel beträgt 40 bis 90 Gew.-%, vorzugsweise 48 bis 60 Gew.-%.

Nachstehend soll das Herstellungsverfahren in allgemeiner Form geschildert werden, wobei das Verfahren nicht auf diese Arbeitsmethode beschränkt sein soll. Es wird eine Lösung von Maleinsäure und Hydrochinon hergestellt. Unter Einleiten von Luft wird die Mischung auf die gewählte Reaktionstemperatur gebracht. Anschliessend wird das Vinylbenzol in der Weise zugegeben, dass die Reaktionstemperatur nicht wesentlich ansteigt. Es können sowohl eine als auch zwei Vinylgruppen zur Reaktion gebracht werden. Nach Beendigung der Reaktion und Abkühlen der Reaktionsmischung wird das ausgefallene Endprodukt abfiltriert und anschliessend umkristallisiert.

Zum Umkristallisieren sind z.B. Acetonitrol oder ein Toluol/Aceton-Gemisch sehr gut geeignet.

Die Ausbeute an Endprodukt (Dianhydrid) kann bis zu 90 Gew.-% betragen.

*Beispiele*

*Beispiel 1*

294 g Maleinsäureanhydrid, 340 ml Toluol und 2,4 g Hydrochinon werden unter Einleiten von 15 l/h Luft (gemessen bei Normalbedingungen: 20°C/1 bar) auf eine Temperatur von 120°C erhitzt und dann langsam mit 156 g Styrol versetzt. Die Styrolzugabe erfolgt in der Weise, dass die Temperatur der Reaktionsmischung konstant bleibt. Nach beendeter Styrolzugabe wird der Ansatz noch 5 h bei 120°C gehalten und nach dem Abkühlen filtriert.

Der Filterrückstand ergibt nach Umkristallisieren aus einem Toluol/Aceton-Gemisch (Vol.-Verhältnis 5 : 1) 351 g (78 Gew.-%) weisses 3,4-Dicarboxy-1,2,3,4-tetrahydro-1-naphthalinbernsteinsäure-dianhydrid («Tetralindianhydrid») mit einem Schmelzpunkt von 202 bis 204°C.

*Beispiel 2*

Die Reaktionsführung analog Beispiel 1 ergibt unter Einleiten von 5 h/l Luft 333 g (74 Gew.-%) «Tetralindianhydrid».

## Patentansprüche

1. Verfahren zur Umsetzung von Maleinsäureanhydrid mit ggf. kernsubstituierten (Di)Vinylbenzolen im Molverhältnis 2 : 0,7 bis 1,3 zu Dianhydrid bei Temperaturen im Bereich von 90 bis 140°C in Gegenwart eines Lösemittels und ggf. substituiertem Hydrochinon unter Durchleiten eines sauerstoffhaltigen Stickstoffstromes durch das Reaktionsgemisch und wobei das Maleinsäureanhydrid in einer 40- bis 90gew.%igen Lösung vorliegt, dadurch gekennzeichnet, dass pro Mol Vinylgruppe 2 bis 20 l/h Luft

(gemessen bei Normalbedingungen: 20°C/1 bar) durchgeleitet werden.

2. Verfahren nach Anspruch 1, dadurch gekennzeichnet, dass 3 bis 10 l/h Luft (gemessen bei Normalbedingungen) durch das Reaktionsgemisch geleitet werden.

## Claims

1. A process for reacting maleic anhydride with (di)vinyl benzenes, which may be substituted in the nucleus, in a molar ratio of 2 : 0.7 to 1.3 to give a dianhydride, at temperatures in the range from 90 to 140°C in the presence of a solvent, and an optionally substituted hydroquinone, while passing an oxygen-containing nitrogen stream through the reaction mixture, and the maleic anhydride being in the form of a 40 to 90% by weight solution, characterized in that 2 to 20 l/hour of air (measured under standard conditions: 20°C/1 bar) are passed through per mol of vinyl groups.

2. A process according to Claim 1, characterized in that 3 to 10 l/hour of air (measured under standard conditions) are passed through the reaction mixture.

## Revendications

1. Procédé de conversion en dianhydride de l'anhydride maléique avec des di(vinylbenzènes éventuellement substitués dans le noyau, dans un rapport molaire de 2 : 0,7 à 1,3, à des températures comprises dans un domaine de 90 à 140°C, en présence d'un solvant, et avec de l'hydroquinone éventuellement substituée, en faisant passer à travers le mélange réactionnel un courant d'azote renfermant de l'oxygène, l'anhydride maléique se présentant dans une solution de 40 à 90% en poids, caractérisé par le fait que l'on fait passer, par mole de groupe vinyle, de 2 à 20 litres d'air par heure (mesuré dans les conditions normales: 20°C/1 bar).

2. Procédé selon la revendication 1, caractérisé par le fait que l'on fait passer par heure, à travers le mélange réactionnel, de 3 à 10 litres d'air (mesuré dans les conditions normales).